# DEMANDE DE BREVET EUROPEEN

(11) **EP 4 207 209 A1**
(43) Date de publication de la demande: **05.07.2023**
(21) Numéro de dépôt: 22150073.9
(22) Date de dépôt: 03.01.2022
(51) Int. Cl.: G16H 20/10

(54) **PROCÉDÉ DE RECOMMANDATION VACCINALE MIS EN OEUVRE PAR UN SYSTÈME INFORMATIQUE**

(71) Demandeur: Syadem, 33000 Bordeaux (FR)
(72) Inventeur: KOECK, Jean-Louis, 33000 BORDEAUX (FR); KOECK, Jean-Denis, 67206 Mittelhausbergen (FR)
(74) Mandataire: Aquinov

(57) **Abrégé**

L'invention concerne un procédé de recommandation vaccinale (1), mis en œuvre par un système informatique (2) comprenant un ensemble de règles de vaccination (VS1) et au moins une interface numérique (NT1), comprenant les étapes suivantes : fourniture d'au moins un ensemble de données de santé d'au moins une personne au moyen de ladite interface numérique (NT1) ; détermination d'au moins un ensemble de recommandations de vaccination par un moteur d'inférence (IM1) à partir dudit ensemble de données de santé fourni et dudit ensemble règles de vaccination (VS1) ; transmission dudit ensemble de recommandations sur ladite interface numérique (NT1).

## Description

La présente invention concerne le domaine technique des services de santé, et vise en particulier un procédé de recommandation vaccinale mis en œuvre par un système informatique.

Les directives de vaccination évoluent constamment et tendent à être plus nombreuses et complexes dans leur application. En effet, toute recommandation de vaccination repose sur la base d'une maximisation du rapport bénéfice/risque pour la personne, nécessitant notamment de caractériser finement les populations éligibles ou celles faisant l'objet de contre-indications. Du fait de la grande diversité desdites caractérisations, il est de plus en plus difficile de comprendre et d'appliquer correctement les recommandations vaccinales, cette difficulté concernant autant les professionnels de santé que les personnes faisant l'objets desdites recommandations. De plus, des nouveaux vaccins voient le jour régulièrement tandis que d'autres tombent en désuétude ou ne sont plus conseillés, sans oublier que les recommandations vaccinales varient selon les pays ou les régions. Par ailleurs, certains vaccins sont mis à jour ou possèdent des variantes destinées, par exemple, aux individus présentant des allergies à certains composés de la formule originale.

Aux points précédemment évoqués s'ajoute le problème du suivi dans le temps des schémas vaccinaux, ces derniers imposant de connaître la date idéale de la prochaine échéance de vaccination. De plus, il est essentiel d'établir des nouvelles recommandations dès lors qu'un changement important s'opère, par exemple, une date de vaccination dépassée, de nouvelles directives de santé publique, etc.

Du fait de cette complexité, le besoin de développer des outils d'aide à la décision adaptés est apparu comme une nécessité ; ont alors vu le jours des systèmes informatiques fournissant un moyen algorithmique de calcul de recommandations vaccinales. Ces systèmes, bien que robustes, présentent un certain nombre de limitations. En effet, les systèmes actuels d'informatisation des recommandations vaccinales sont basés sur un encodage en brut des règles de vaccination, ce qui ne garantit pas un résultat sans recommandations contradictoires et limite la possibilité de mettre à jour facilement et régulièrement les recommandations de vaccination définies dans le système informatique par des personnes sans expertise informatique, notamment des experts de la vaccination. À cette limitation s'ajoute l'incapacité des systèmes existants de reconnaître et traiter la pluralité d'appellations courantes des vaccins employés contre une même maladie.

La présente invention se place ainsi dans ce contexte et cherche à résoudre l'ensemble des inconvénients précités, en proposant un procédé de recommandation vaccinale qui permette de définir, pour un utilisateur particulier, notamment une personne, un ensemble de recommandations personnalisées de vaccinations pour cet utilisateur, basé sur toutes les règles de vaccination pertinentes pour cet utilisateur, et dans lequel il soit possible de déployer rapidement et aisément une nouvelle règle de vaccination.

À ces fins, l'invention a pour objet un procédé de recommandation vaccinale, mis en œuvre par un système informatique et comprenant un ensemble de règles de vaccination et au moins une interface numérique, comprenant les étapes suivantes :
a. Fourniture d'au moins un ensemble de données de santé d'au moins une personne au moyen de ladite interface numérique ;
b. Détermination d'au moins un ensemble de recommandations de vaccination par un moteur d'inférence à partir dudit ensemble de données de santé fourni et dudit ensemble de règles de vaccination ;
c. Transmission dudit ensemble de recommandations de vaccination déterminé à ladite interface numérique.

On comprend ainsi que grâce à l'invention, on procède d'une part à la délivrance d'un ensemble de recommandations pertinentes, notamment non contradictoires, de vaccination pour une personne donné, dans la mesure où ces recommandations sont basées sur la récolte d'informations relatives à cette personne via l'interface numérique. L'interface numérique pourra indifféremment être une application logicielle ou un site web accessible depuis un téléphone intelligent, une tablette numérique, un ordinateur. Dans ce cas, un utilisateur du système informatique, pouvant par exemple être un expert de la vaccination, un professionnel de santé ou la personne elle-même, peut fournir lui-même les données de santé de la personne au système informatique. En variante, l'interface numérique pourra être une interface permettant à un système d'information tiers de fournir des données de santé d'une personne au système informatique. On pourra ainsi concevoir que le système d'information d'un établissement de santé puisse communiquer les données d'une personne, par exemple issues de son dossier médical partagé, au système informatique. En outre, l'utilisation d'un moteur d'inférence permet de supprimer, d'ajouter ou de mettre à jour une règle de vaccination dans l'ensemble de règles de vaccination, lequel forme une base de règles ou une base de connaissance du moteur d'inférence, de sorte que cette modification se répercute aisément et rapidement sur la détermination de l'ensemble de recommandations de vaccination d'une personne quelconque sans qu'il soit nécessaire de procéder à une modification profonde, voire une refonte, du code logiciel mis en œuvre par le système informatique.

Dans la présente invention, on entend par « calendrier de vaccination », notamment un ensemble d'informations portant sur :
a. au moins une information relative à une maladie donnée,
b. au moins une première échéance d'administration d'un vaccin contre ladite maladie donné,
c. au moins un statut vaccinal relatif à l'administration dudit vaccin donné contre ladite maladie donnée à la dite échéance.

On pourra prévoir notamment qu'un calendrier de vaccination puisse également contenir des informations relatives à plusieurs schémas vaccinaux associés à des recommandations de vaccination contre différentes maladies, ainsi que l'ensemble des échéances d'administrations de vaccination associées à chacune desdites recommandations.

Dans la présente invention, on entend par « recommandation de vaccination » notamment une recommandation d'administration d'un vaccin contre une maladie donnée à une personne répondant à un ensemble de critères donnés, notamment établie par une institution compétente. On entend notamment par « ensemble de critères donnés » un ou plusieurs critères choisis parmi le métier, l'âge, le sexe, l'état de santé, l'environnement, l'exposition à des animaux ou à des matières biologiques. Ladite recommandation de vaccination pourra, le cas échéant, identifier une date de vaccination, une contre-indication médicale ou encore un cas exceptionnel requérant une expertise humaine. On pourra prévoir qu'une recommandation de vaccination contienne une justification de ladite recommandation de vaccination, ciblée pour la personne compte tenu de l'ensemble desdits critères donnés, ladite justification contenant par exemple des références aux règles employées par le moteur d'inférence pour aboutir à la recommandation de vaccination et éventuellement des sources bibliographiques de ces règles employées. Cette justification donne ainsi la possibilité à l'utilisateur de connaître les fondements décisionnels des recommandations personnalisées retournées par le moteur d'inférence.

Dans la présente invention, on entend par « règle de vaccination » notamment une formule logique interprétable par un ordinateur et traduisant au moins une recommandation de vaccination.

Dans la présente invention, on entend par « questionnaire » notamment une séquence de requêtes d'information devant être complétées par la personne. Il pourra notamment s'agir d'une séquence ordonnée de questions, présentée en bloc ou de façon séquentielle, et visant à récolter des informations concernant des maladies connues présentes ou passées, des allergies, un contact avec des publics sensibles comme des enfants, des personnes âgées, des femmes enceintes ou des personnes immunodéprimées, une exposition à des animaux, la profession exercée, un pays de séjour, un statut de grossesse, la composition familiale ou des projets de voyage.

Dans la présente invention, on entend par « profil de la personne » notamment un ensemble d'informations personnelles associées à un utilisateur, notamment par référencement d'un identifiant de ladite personne dans une base de données. Ainsi, un profil de la personne pourra par exemple contenir notamment des informations sur la date et le lieu de naissance, le numéro de téléphone, l'adresse électronique, l'adresse postale, le numéro national d'identité, le numéro de carnet de vaccination, le numéro de passeport ou le numéro de visa.

Dans la présente invention, on entend par « règle de précédence » notamment une formule logique traduisant un critère d'ordre sur la priorité d'un ensemble de recommandations de vaccination.

Dans la présente invention, en entend par « interface numérique » notamment un moyen d'échange de données de type terminal numérique, application logicielle, site web, ou interface avec un système informatique tiers, permettant de communiquer avec le système informatique pour fournir ledit ensemble de données de santé et récolter l'ensemble de recommandations déterminées.

Dans un exemple de réalisation de l'invention, l'étape de fourniture dudit ensemble de données de santé comporte la fourniture d'un profil de la personne au moyen de ladite interface numérique.

Avantageusement, l'étape de fourniture dudit ensemble de données de santé de la personne comporte la fourniture d'un historique de vaccination de la personne au moyen de ladite interface numérique. Grâce à la fourniture d'un historique de vaccination de la personne, il est possible de connaître les vaccins déjà administrés à ladite personne ainsi que leurs dates d'administration. Par conséquent, pour tout vaccin déjà administré, il est aisé d'établir la date de rappel ou de dose supplémentaire suivante, ainsi que d'identifier les vaccins qui seraient potentiellement à faire. Par ailleurs, l'historique de vaccination permet d'éviter d'établir une recommandation de vaccination pour un vaccin déjà administré à la personne.

Dans un mode de réalisation de l'invention, l'étape de fourniture dudit ensemble de données de santé comporte la fourniture d'une pluralité de réponses apportées à un questionnaire par la personne au moyen de ladite interface numérique. Grâce à la fourniture de ladite pluralité de réponses le procédé pourra affiner la pertinence des recommandations qui seront retournées, notamment en évitant de conseiller un vaccin inutile, compte tenu du profil de la personne, ou en adaptant des recommandations générales à une situation personnelle de la personne.

Dans un mode de réalisation de l'invention, l'étape de fourniture dudit ensemble de données comporte la fourniture d'un état d'au moins un schéma vaccinal associé à un vaccin donné et/ou une maladie donnée de la personne au moyen de ladite interface numérique. Ledit état pourra par exemple être encodé sous la forme d'un code barre, code QR, hyperlien ou tout autre mode d'encodage possible.

Avantageusement, l'étape de détermination d'au moins un ensemble de recommandations de vaccination par le moteur d'inférence est renouvelée pour chaque nouvel ensemble de données de santé fourni par la personne au moyen de ladite interface numérique. Grâce au renouvèlement desdites étapes le procédé permet de maximiser la pertinence desdites recommandations de vaccination en tenant compte des informations les plus récentes.

Dans un mode de réalisation, ledit ensemble de règles de vaccination comporte des règles logiques, temporelles ou arithmétiques formant une base de règles ou de connaissances du moteur d'inférence. Compte tenu de l'importance du nombre de règles de vaccination pouvant s'appliquer à une même maladie, notamment du fait des différences de situation personnelle, médicale ou professionnelle chez les personnes, une approche traditionnelle par un arbre de décision est peu judicieuse tant la combinatoire est complexe. L'utilisation d'un moteur d'inférence basé sur un tel ensemble de règles permet de simplifier la mise en œuvre et l'exploitation du procédé de recommandation. En effet, le moteur d'inférence peut ainsi obtenir plusieurs recommandations de vaccination pour une même maladie en appliquant différentes règles, un arbitrage pouvant ensuite être conduit pour déterminer la meilleure recommandation de vaccination.

Dans un mode de réalisation le moteur d'inférence est un moteur d'inférence à chaînage avant. Cette caractéristique confère à ce type de moteurs d'inférence l'avantage de pouvoir fournir des résultats rapidement, ce qui offre un gain de temps de calcul sur lesdites recommandations de vaccination.

Dans un autre mode de réalisation, le moteur d'inférence est un moteur d'inférence à chaînage arrière. Cette caractéristique structurelle confère à ce type de moteurs d'inférence l'avantage de permettre une formulation compacte et élégante des règles de calcul, en l'occurrence des règles de vaccination.

En variante, le moteur d'inférence est un moteur d'inférence à chaînage mixte. Ce type de moteur d'inférence se caractérise par une syntaxe compacte et élégante des règles de calcul tout en renvoyant les résultats des recommandations avec un faible temps de calcul.

Dans un mode de réalisation de l'invention, l'étape de détermination d'un ensemble de recommandations de vaccination comprend une sous-étape de détermination, par le moteur d'inférence, d'un ensemble primaire de recommandations de vaccination à partir dudit ensemble de données de santé fourni et de l'ensemble de règles de vaccination et une sous-étape de résolution de conflits des recommandations dudit ensemble primaire de recommandations pour former ledit ensemble de recommandations de vaccination. On comprend alors que lors de l'application des règles de vaccinations audit ensemble de données de santé, l'ensemble de recommandations de vaccination peut contenir des recommandations conflictuelles entre elles ou par rapport auxdites informations de santé. L'étape de résolution de conflits permet ainsi d'arbitrer entre ces recommandations afin de fournir un ensemble de recommandations adapté à la personne, y compris dans des cas complexes.

Avantageusement, la sous-étape de résolutions de conflits comporte l'organisation dudit ensemble primaire en jeux de recommandations visant une même maladie, et pour chaque jeu comportant au moins deux recommandations distinctes, le tri des recommandations dudit jeu selon au moins une règle de précédence, dans lequel ledit ensemble de recommandations de vaccination comporte uniquement la première recommandation dudit jeu trié. Grâce au tri par règle de précédence s'établit un ordre total sur l'ensemble des recommandations. Il est ainsi possible de comparer, selon un critère de prévalence donné, deux recommandations quelconques et de les classer par rapport au dit critère.

Avantageusement, une règle de précédence définit un critère d'ordre total où une exception prévaut sur une contre-indication, une contre-indication prévaut sur une vaccination à faire et la vaccination à faire dont la prochaine échéance est la plus courte prévaut sur toute autre vaccination à faire. Grâce auxdites règles de précédence et dudit ordre total on arrive à minimiser un risque médical pour la personne.

Dans un mode de réalisation de l'invention, le système informatique comporte une base de données de calendriers de vaccination, le procédé comportant une étape de sélection d'un calendrier associé à la personne dans ladite base de données, de mise à jour dans ladite base de données dudit calendrier sélectionné à partir dudit ensemble de recommandations déterminé. Le calendrier de vaccination déterminé à partir de cet ensemble de recommandations est ainsi personnalisé pour cette personne et peut être mis à jour afin de fournir un suivi exact dans le temps des schéma vaccinaux. Dans un exemple particulier, ledit calendrier sélectionné et mis à jour peut être affiché sur ladite interface numérique.

Dans un mode de réalisation de l'invention, en l'absence d'un calendrier associé à la personne dans la base de données de calendriers de vaccination, le procédé comporte une étape d'instanciation dans ladite base de données, d'un calendrier associé à la personne.

Dans un mode de réalisation de l'invention, l'étape de mise à jour dudit calendrier sélectionné comporte, pour chaque recommandation vaccinale dudit ensemble de recommandations de vaccination déterminé, une modification ou un ajout d'un statut vaccinal pour la maladie concernée par ladite recommandation vaccinale et une modification ou un ajout d'une première échéance d'administration d'un vaccin contre ladite maladie concernée par ladite recommandation vaccinale. Éventuellement, on pourra prévoir d'ajouter ou de modifier l'une des informations suivantes : date provisoire de vaccination, date effective de vaccination, délai minimal et maximal de vaccination et nombre de doses. Grâce à ladite étape de mise à jour dudit calendrier sélectionné, on s'assure de garder un registre des schémas vaccinaux et de leur suivi. Avantageusement, ledit statut permet à la personne d'agir correctement à tout moment, y compris dans le cas d'une date recommandée de vaccination dépassée, auquel cas de nouvelles recommandations peuvent s'appliquer.

Avantageusement, le système informatique comporte une interface numérique d'ajout apte à recevoir une recommandation de vaccination, et agencée pour encoder ladite recommandation vaccinale en une règle de vaccination interprétable par le moteur d'inférence et pour ajouter ladite règle de vaccination à l'ensemble de règles de vaccination, caractérisé en ce qu'il comporte une étape préalable de production du premier ensemble de règles de vaccination comportant la fourniture d'une pluralité de recommandations de vaccination à ladite interface numérique d'ajout. Grâce à ladite interface numérique d'ajout s'effectue l'encodage de toute recommandation de vaccination souhaitée, particulièrement en vue de son utilisation par le moteur d'inférence, de manière simple et sans avoir recours à l'écriture en brut de ladite recommandation dans un langage informatique, permettant ainsi à des personnes sans connaissance de programmation de compléter et mettre à jour ledit premier ensemble de règles de vaccination.

Dans un mode de réalisation de l'invention, le système informatique est apte à transmettre ou recevoir, via l'interface numérique, un ensemble d'informations relatives aux calendriers de vaccinations d'une pluralité de personnes stockés dans la base de données de calendriers de vaccinations avec un système numérique externe. Le système numérique externe pourra par exemple être un système de gestion de stock de vaccins, notamment d'un hôpital, d'une pharmacie, d'un centre de vaccination, d'un système de gestion épidémiologique ou d'un système d'information médical et centralisé.

Par exemple, le système informatique pourra transmettre un ensemble d'informations comportant des informations sur la couverture vaccinale de ladite pluralité de personnes ou des informations relatives à une distribution, notamment quantitative, spatiale ou temporelle, des vaccins administrés à ladite pluralité de personnes. Le système informatique peut ainsi fournir des données statistiques permettant par exemple de contrôler une opération de vaccination contre une maladie donnée.

En variante, le système informatique pourra transmettre un ensemble d'informations comportant des informations relatives à un nombre de doses d'un même vaccin ou d'un groupe de vaccins administrés à ladite pluralité de personnes ou à un nombre de doses d'un même vaccin ou d'un groupe de vaccins devant être administrés à ladite pluralité de personnes. Le système informatique peut ainsi permettre à un établissement de santé de prévoir un renouvellement de stock de doses de vaccin contre une maladie donnée.

En variante encore, le système informatique pourra transmettre un ensemble d'informations comportant des informations relatives à ladite pluralité de personnes, laquelle pourra être une pluralité de personnes auxquels auront été administrés une dose d'un vaccin, l'administration de cette dose de vaccin auxdites personnes de cette pluralité présentant au moins un caractère commun, notamment un numéro identique de lot d'appartenance des vaccins administrés, un mode d'administration identique desdits vaccins, un producteur identique desdits vaccins administrés, ou encore un lieu ou une date d'administration identique.

Avantageusement, le système informatique peut être agencé pour transmettre une notification à un terminal distant, notamment un téléphone intelligent, d'un utilisateur en fonction d'une comparaison d'une date donnée, par exemple la date du jour, à une date de vaccination prévue dans le calendrier de vaccination dudit utilisateur stocké dans la base de données. Ladite notification pourra par exemple être un courriel électronique, un message texte, ou une notification visuelle ou sonore émise par une application installée sur ledit terminal distant.

Dans un mode de réalisation, ledit système informatique peut comporter une base de données de vaccins, le moteur d'inférence déterminant ledit ensemble de recommandations de vaccination à partir dudit ensemble de données de santé fourni, de l'ensemble de règles de vaccination et de la base de données de vaccins.

Avantageusement, ledit ensemble de recommandations de vaccination déterminé est généré à partir d'un ensemble de sources de données de vaccins et d'une ontologie pivot définissant des classes dérivées d'une ou plusieurs ontologies de vaccins et des relations entre les classes, le moteur d'inférence déterminant ledit au moins un ensemble de recommandations de vaccination à partir dudit ensemble de données de santé fourni, de l'ensemble de règles de vaccination et de la base de données de vaccins. Une « ontologie pivot » désigne une ontologie contenant au moins deux jointures, vers au moins deux autres ontologies indépendantes. Elle forme ainsi une composition réfléchie de plusieurs autres ontologies du même domaine de connaissance afin de faciliter et de mieux représenter une notion, un concept au travers de plusieurs définitions formelles. Un procédé préalable peut être appliqué afin de choisir et de (re)former les définitions recherchées. Cet aspect pivot permet de rapidement relier des données annotées par ces différentes ontologies séparément qui n'avaient pas pour but d'être interopérables à l'origine.

Dans un mode de réalisation, l'ontologie pivot peut comprendre trois classes principales : une classe principale « Vaccin » ; une classe principale « Valence » et une classe principale « Maladie cible ».

Les sources de données peuvent comprendre des sources de données prévues pour tenir compte des différents acteurs du vaccins, comme par exemple :
a. les laboratoires pharmaceutiques, chercheurs, innovateurs et producteurs de vaccins ; les sociétés commerciales assurant la diffusion de vaccins ; les organismes régulateurs comme l'Agence nationale pour la sécurité du médicament et des produits de santé (ANSM) ou l'Agence européenne des médicaments (EMA), qui autorisent la mise sur le marché des vaccins; la Haute Autorité de Santé (HAS), qui élabore les recommandations vaccinales ; le ministère de la santé, qui publie des directives sur la mise en œuvre des recommandations vaccinales.
b. les professionnels de santé et établissements de santé prescripteurs ;
c. les lieux d'acquisition des vaccins ;
d. les pharmacies d'officines et établissements de santé ;
e. des bases de données générées par des organismes privés (Thériaque, Vidal, Claude Bernard, mutuelles, assurances) ;
f. des bases de données publiques : assurance maladie, ANSM, en liaison avec la HAS ;
g. des sources de données non structurées générées par les organismes et professionnels de santé comprenant des résumés des caractéristiques des produits ;

L'invention a également pour objet un système informatique agencé pour mettre en œuvre un procédé de recommandation vaccinale selon l'invention.

La présente invention est maintenant décrite à l'aide d'exemples uniquement illustratifs et nullement limitatifs de la portée de l'invention, et à partir des dessins annexés, dessins sur lesquels les différentes figures représentent :

[Fig. 1] représente, schématiquement et partiellement, un procédé de recommandation vaccinale selon un mode de réalisation de l'invention ;

[Fig. 2] représente, schématiquement et partiellement, un exemple de système informatique mettant en œuvre un procédé de recommandation vaccinale selon un mode de réalisation de l'invention.

Dans la description qui suit, les éléments identiques, par structure ou par fonction, apparaissant sur différentes figures conservent, sauf précision contraire, les mêmes références.

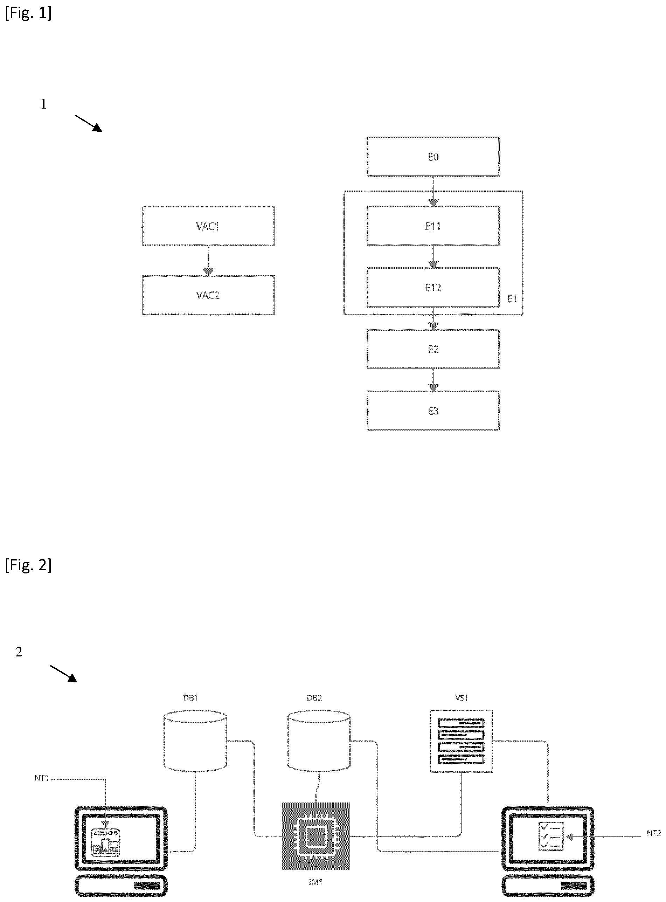

On a représenté en [Fig.1] un procédé de recommandation vaccinale 1 selon un mode de réalisation de l'invention. On a représenté en [Fig. 2] un système informatique 2 mettant en œuvre le procédé de recommandation vaccinale 1 de la [Fig. 1] et comportant une première base de données DB1 contenant des calendriers de vaccination, une deuxième base de données DB2 de vaccins, un ensemble VS1 de règles de vaccination, un moteur d'inférence IM1 et deux interfaces numériques NT1 et NT2.

On va décrire dans un premier temps le fonctionnement du procédé au travers de la succession d'étapes qui le composent.

Le procédé de recommandation vaccinale 1 comporte une étape préliminaire VAC1 de création de la base de données de vaccins DB2, ladite base de données de vaccins étant générée à partir d'un ensemble de sources de données de vaccins et d'une ontologie pivot définissant des classes dérivées d'une ou plusieurs ontologies de vaccins et des relations entre les classes. Avantageusement, le contenu de la base de données de vaccins DB2 peut être crée, mis-à-jour et supprimé au travers de l'interface numérique NT2. L'utilisateur, notamment un expert de la vaccination, rentre un nouvel élément dans ladite base de données en inscrivant un code d'identification unique d'un vaccin et en lui associant, d'une part, une pluralité de nomenclatures incluant par exemple le nom commercial du vaccin, le nom de la maladie ciblée, la valence dudit vaccin ; et d'autre part, des informations relatives à l'utilisation dudit vaccin incluant par exemple le nombre de doses prévues dans un schéma vaccinal complet, la composition dudit vaccin, le mode d'administration dudit vaccin, un ensemble de contre-indications ainsi que des informations sur les effets indésirables dudit vaccin.

Le procédé 1 comporte ensuite une étape VAC2 de création de l'ensemble de règles de vaccination VS1, ladite base de données étant générée à partir d'un ensemble de sources de données de recommandations de vaccination, notamment des recommandations de vaccination établies par un organisme de santé, et d'une ontologie pivot définissant des classes dérivées d'une ou plusieurs ontologies de recommandations de vaccination et des relations entre les classes. Avantageusement, le contenu de l'ensemble de règles de vaccination VS1 peut être crée, mis à jour et supprimé au travers de l'interface numérique NT2. L'utilisateur, notamment un expert de la vaccination, rentre un nouvel élément dans ledit ensemble de règles de vaccination VS1 en répondant à une série de questions ou en sélectionnant une série d'éléments présentés avantageusement sous forme de cases à cocher, menus déroulants, questionnaires, boutons, barres de défilement ou encore de fenêtres de saisie. À la suite de la saisie de ladite recommandation de vaccination par l'utilisateur, l'interface numérique NT2 traduit automatiquement les éléments en une règle de vaccination et la sauvegarde dans l'ensemble de règles de vaccination VS1. Dans le cas où l'utilisateur souhaite modifier le contenu d'une règle de vaccination, l'interface numérique NT2 est apte à établir le processus inverse et permet de traduire toute règle de vaccination en éléments de ladite interface numérique caractérisés en ce qu'ils sont modifiables directement par l'utilisateur. Ladite interface numérique NT2 permet ainsi à l'utilisateur de définir simplement et confortablement des règles de vaccination sans avoir à les encoder lui-même en langage informatique, il n'est donc pas nécessaire que l'utilisateur dispose des connaissances de programmation afin de créer, mettre à jour ou supprimer des règles de vaccination.

La base de données de calendriers de vaccination DB1 contient des calendriers de vaccination, repérés par un code d'identification unique. Lesdits calendriers de vaccination sont aptes à contenir des informations de date, heure et lieu ainsi que des informations concernant des vaccins comme le nom, la maladie ciblée, le producteur du vaccin, le nombre de doses, l'intervalle de temps (notamment minimal, parfois maximal) entre deux vaccins ou entre deux doses et des statuts vaccinaux. Avantageusement, le contenu de la base de données de calendriers de vaccination DB1 peut être visualisé, crée, mis-à-jour et supprimé au travers de l'interface numérique NT1. L'utilisateur, notamment une personne, peut par exemple accéder à son calendrier de vaccination à l'aide de ladite interface numérique NT1, notamment en se connectant à une application logicielle ou un site internet. Il peut alors savoir, à l'aide de son calendrier de vaccination, si des vaccinations sont à faire, si elles le sont prochainement ou pas, ainsi que connaître l'ensemble des échéances desdites vaccinations.

Lors de la première utilisation de l'application logicielle ou du site internet, l'utilisateur peut être invité, via un formulaire, à créer un compte en indiquant un identifiant et un mot de passe. En réponse à cette création de compte, le procédé comporte une étape de création ou d'instanciation d'un calendrier de vaccination (non représentée), dans la base de données DB1, associé à un utilisateur, à son identifiant et auquel est attribué un nouveau code d'identification unique.

Le procédé de recommandation vaccinale 1 comporte ensuite une étape E0 de fourniture d'au moins un ensemble de données de santé par un utilisateur au moyen de l'interface numérique NT1. Ledit ensemble de données de santé est constitué notamment d'un profil, d'un historique de vaccination ou des réponses à des questionnaires.

Le procédé 1 comporte ensuite une étape E1 de détermination d'au moins un ensemble de recommandations de vaccination, par le moteur d'inférence IM1, à partir de l'ensemble de données de santés fournies à l'étape E0 et des règles de vaccinations de l'ensemble VS1. Le moteur d'inférence IM1 applique l'ensemble des règles de recommandations constituant l'ensemble de règles de vaccination VS1 en faisant correspondre les informations apportées à l'étape E0 aux éléments logiques pertinents dans les règles de vaccination. Par exemple, un élément logique « âge de l'utilisateur » apparaissant dans toute règle de vaccination sera remplacé par l'âge indiquée par l'utilisateur à l'étape E0. Ledit ensemble de recommandations de vaccination, obtenu à l'issue de l'étape E1, est transmis à l'interface numérique NT1 dans une étape E2.

L'étape E1 est composée de deux sous étapes : E11 et E12. La sous-étape E11 correspond à la détermination par le moteur d'inférence IM1 d'un ensemble primaire de recommandations de vaccination. Étant donné le profil particulier de l'utilisateur, ledit ensemble primaire de recommandations de vaccination est susceptible de contenir des conflits dans les recommandations calculées en première instance. C'est notamment le cas des vaccins qui pourraient à la fois et pour le même utilisateur être conseillés d'après une recommandation, par exemple du fait de la situation professionnelle de la personne, et déconseillés d'après une autre, par exemple étant donné une contre-indication médicale, notamment une grossesse en cours ou une maladie connue. Afin de résoudre cette problématique, le procédé comporte une sous-étape E12 correspondant à la résolution de conflits dudit ensemble primaire de recommandations au travers de règles de précédence dans lesquelles une exception prévaut sur une contre-indication, une contre-indication prévaut sur une vaccination à faire et la vaccination à faire dont la prochaine échéance est la plus courte prévaut sur toute autre vaccination à faire.

Le procédé comporte ensuite une étape E3 de sélection du calendrier de vaccination crée initialement et repéré par le code d'identification unique associé à un utilisateur, et de mise à jour dudit calendrier en appliquant les recommandations de vaccination obtenues à l'issue de l'étape E1. Ce calendrier mis à jour est alors affiché sur l'interface numérique NT1.

La mise à jour dudit calendrier comporte la création des échéanciers vaccinaux nécessaires pour satisfaire auxdites recommandations de vaccination, comportant notamment des dates idéales de vaccination pour chaque vaccin recommandé. À chaque échéance de vaccination est associé un statut « vaccination à faire ». On pourrait concevoir d'associer d'autres statuts déterminés, par exemple en comparant l'échéance de vaccination à la date du jour, comme, « vaccination à faire prochainement », « vaccination à faire au plus vite », « date de vaccination dépassée », « vaccination à jour », « schéma vaccinal complet ». Ledit statut permet à l'utilisateur de suivre l'ensemble de ses schémas vaccinaux et par ce biais d'apporter une aide dans l'organisation de l'utilisateur afin d'éviter des schémas vaccinaux incorrects qui pourraient porter préjudice à l'utilisateur, notamment lorsqu'une date de vaccination est dépassée.

Le statut vaccinal du calendrier de vaccination a notamment la propriété de changer en fonction de la distance temporelle entre la date du jour et la date idéale de vaccination. Ainsi, le statut peut évoluer de « vaccination à faire » à « vaccination à faire prochainement », « vaccination à faire au plus vite » ou encore à « date de vaccination dépassée ». À la suite d'une modification quelconque des informations de santé de l'utilisateur, par exemple des vaccinations nouvellement réalisées, la découverte de nouvelles maladies, le changement de métier, entre autres, entraînent un nouveau calcul de l'ensemble de recommandations de vaccination par le moteur d'inférence ainsi que des modifications des informations du calendrier de vaccination associé audit utilisateur, notamment des informations portant sur le nombre de doses, le type de vaccin et le statut vaccinal.

On notera que les étapes E0 et E1, de même que les étapes E2 et E3, peuvent être renouvelées à chaque reconnexion de l'utilisateur à son compte, via l'application logicielle ou le site web, de sorte que le calendrier de vaccination associé à cet utilisateur soit mis à jour, au travers du moteur d'inférence IM1, dès que l'utilisateur souhaite apporter de nouvelles données médicales ou préciser une situation de santé à laquelle il est confronté.

La description qui précède explique clairement comment l'invention permet d'atteindre les objectifs qu'elle s'est fixée, à savoir fournir automatiquement à un utilisateur un ensemble de recommandations de vaccination adaptées à sa personne en tenant compte des informations de santé dudit utilisateur et d'un ensemble de règles de vaccination, ainsi que de lui offrir la possibilité d'un suivi dynamique dans le temps de ses schémas vaccinaux, en permettant de déterminer l'ensemble des recommandations vaccinales qui s'appliquent à l'utilisateur par calcul sur un moteur d'inférence et de résoudre d'éventuels conflits dans les recommandations en appliquant, si nécessaire, des règles de précédence définies par un critère de prévalence, et en constituant un calendrier numérique permettant de planifier les schémas vaccinaux correspondants ainsi que de recevoir des alertes et, en cas de besoin, de mettre à jour le contenu dudit calendrier.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourra par exemple envisager de relier le système de recommandation vaccinale avec des informations issues de documents de voyage, notamment des passeports vaccinaux.

## Revendications

1. Procédé de recommandation vaccinale (1), mis en œuvre par un système informatique (2) comprenant un ensemble de règles de vaccination (VS1), et au moins une interface numérique (NT1), comprenant les étapes suivantes :
a. (E0) Fourniture d'au moins un ensemble de données de santé d'au moins une personne au moyen de ladite interface numérique (NT1) ;
b. (E1) Détermination d'au moins un ensemble de recommandations de vaccination par un moteur d'inférence (IM1) à partir dudit ensemble de données de santé fourni et dudit ensemble de règles de vaccination (VS1) ;
c. (E2) Transmission dudit ensemble de recommandations de vaccination déterminé à ladite interface numérique (NT1).

2. Procédé (1) selon la revendication précédente dans lequel l'étape (E0) de fourniture dudit ensemble de données de santé comporte la fourniture d'un profil de la personne au moyen de ladite interface numérique (NT1).

3. Procédé (1) selon l'une des revendications précédentes, dans lequel l'étape (E0) de fourniture dudit ensemble de données de santé comporte la fourniture d'un historique de vaccination de la personne au moyen de ladite interface numérique (NT1).

4. Procédé (1) selon l'une des revendications précédentes, dans lequel l'étape de fourniture (E0) dudit ensemble de données de santé comporte la fourniture d'une pluralité de réponses apportées, par la personne, à un questionnaire au moyen de ladite interface numérique (NT1).

5. Procédé (1) selon l'une des revendications précédentes, dans lequel l'étape (E1) de détermination d'au moins un ensemble de recommandations de vaccination par le moteur d'inférence (IM1) est renouvelée pour chaque nouvel ensemble de données de santé de la personne fourni au moyen de ladite interface numérique (NT1).

6. Procédé (1) selon l'une des revendications précédentes dans lequel le moteur d'inférence (IM1) est à chaînage avant ou chaînage arrière ou chaînage mixte.

7. Procédé (1) selon l'une des revendications précédentes dans lequel l'étape (E1) de détermination d'un ensemble de recommandations de vaccination comprend une sous-étape (E11) de détermination, par le moteur d'inférence (IM1), d'un ensemble primaire de recommandations de vaccination à partir dudit ensemble de données de santé fourni et de l'ensemble de règles de vaccination (VS1) et une sous-étape (E12) de résolution de conflits des recommandations dudit ensemble primaire de recommandations pour former ledit ensemble de recommandations de vaccination.

8. Procédé (1) selon la revendication précédente, dans lequel la sous-étape (E12) de résolution de conflits comporte l'organisation dudit ensemble primaire en jeux de recommandations visant une même maladie, et pour chaque jeu comportant au moins deux recommandations distinctes, le tri des recommandations dudit jeu selon au moins une règle de précédence, dans lequel ledit ensemble de recommandations de vaccination comporte uniquement la première recommandation dudit jeu trié.

9. Procédé (1) selon l'une des revendications précédentes, dans lequel le système informatique (2) comporte une base de données (DB1) de calendriers de vaccination, le procédé comportant une étape (E3) de sélection d'un calendrier associé à la personne dans la base de données (DB1) et de mise à jour dans la base de données (DB1) dudit calendrier sélectionné à partir dudit ensemble de recommandations déterminé.

10. Procédé (1) selon la revendication précédente, **caractérisé en ce que**, en l'absence d'un calendrier associé à la personne dans la base de données (DB1), le procédé comporte une étape d'instanciation, dans la base de données (DB1), d'un calendrier associé à la personne.

11. Procédé (1) selon la revendication précédente, dans lequel l'étape (E3) de mise à jour dudit calendrier sélectionné comporte, pour chaque recommandation vaccinale dudit ensemble de recommandations de vaccination sélectionnées, une modification dudit statut, date, délai, nombre de doses.

12. Procédé (1) selon l'une des revendications précédentes, dans lequel le système informatique (2) comporte une interface numérique (NT2) d'ajout apte à recevoir une recommandation de vaccination, et agencée pour encoder ladite recommandation vaccinale en une règle de vaccination interprétable par le moteur d'inférence (IM1) et pour ajouter ladite règle de vaccination à l'ensemble de règles de vaccination (VS1), **caractérisé en ce qu'**il comporte une étape préalable de production de l'ensemble de règles de vaccination (VS1) comportant la fourniture d'une pluralité de recommandations de vaccination et d'une interface numérique d'ajout (NT2).
